# EUROPEAN PATENT SPECIFICATION

(11) **EP 0 814 773 B1**
(45) Date of publication and mention of the grant of the patent: **25.07.2001**
(21) Application number: 96908089.4
(22) Date of filing: 16.03.1996
(51) Int. Cl.: A61K 9/00

(54) **PECTIN LIQUID PHARMACEUTICAL COMPOSITIONS**
FLÜSSIGE PHARMAZEUTISCHE ZUSAMMENSETZUNGEN MIT PEKTIN
COMPOSITIONS PHARMACEUTIQUES LIQUIDES A BASE DE PECTINE

(30) Priority: 17.03.1995 GB 9505368; 05.04.1995 GB 9507037
(43) Date of publication of application: 07.01.1998
(73) Proprietor: The Boots Company PLC, Nottingham, Nottinghamshire NG2 3AA (GB)
(72) Inventor: COX, Gillian, Nottingham NG2 3AA (GB)
(74) Representative: Thacker, Michael Anthony
(86) International application number: EP9601205
(87) International publication number: WO9629055

(56) References cited:
- EP-A- 0 286 085
- GB-A- 2 283 172

## Description

The present invention relates to pharmaceutical compositions used to treat disorders of the digestive system. In particular, the invention relates to liquid preparations for use in the prevention of gastric reflux.

Gastric reflux is the passage of small amounts of highly acidic gastric juice and bile acids from the stomach into the lower part of the oesophagus. This causes the pain of heartburn and can inflame or damage the oesophagus. Gastric reflux may be symptomatic of a variety of disorders such as ulcers or biliary conditions.

Preparations which are known to treat gastric reflux comprise a substance which when exposed to the acid contents of the stomach forms a gel which floats on the surface of the stomach contents as a raft due to a lower bulk density. The lower bulk density is achieved by entrapment of gas bubbles within the gel structure. The gas is generated by a material which produces gas in the acidic conditions of the stomach as the gel is being formed, for example a carbonate or bicarbonate which produces carbon dioxide. The gel raft acts as a physical barrier to gastric reflux. The raft, which is located in the upper part of the stomach (fundus), also protects the oesophagus if reflux does occur as on reflux the raft contacts the oesophagus before the gastric juices, providing some further protection.

Examples of such products include Gaviscon® (Reckitt and Colman). This is described in GB 1524740 as a liquid or tablet formulation which forms an alginate raft to suppress gastric reflux. In practice, the raft forms relatively slowly. A similar alginate raft product Algicon® is described in WO 85/04806 (Rorer). This document is directed towards producing a composition of low sodium content. Other raft forming products currently marketed [e.g. Gastrocote® (Boeringer Mannheim), BiSoDol® (Whitehall) and Gastron® (Sanofi Winthrop)] also use alginates as the gel component.

Use of alginates as the gel forming component has various disadvantages. Alginates are extracted from various different brown seaweeds of the class Phaeophyceae which are found of the coast of a limited number of countries throughout the world. This leads to an uncertain source of supply. There is wide variation in the properties of the alginates isolated from different seaweeds due to the different ratio of mannuronic acid to guluronic acid monosaccharides in the alginate polysaccharide isolated from each species.

Polysaccharides which are particularly suitable for use in raft compositions are pectins. For a discussion and an outline of the nature of pectins, the reader is referred to Kirk-Othmer's Encyclopaedia of Chemical Technology (1992) 4th Ed., Vol. 4, 943-944, published by John Wiley & Sons. Pectins are naturally occurring polysaccharides located in the cell walls of all plant tissues which can be extracted from higher plant sources. Pectins comprise a linear chain of C₁₋₄ linked d-galactopyranosyluronic acid units, which makes the pectin molecule an d-galacturonan (a poly-d-galactopyranosyluronic acid) or an d-galacturonoglycan.

Pectins are divided according to the percentage of carbonyl groups esterified with methanol (known as the degree of esterification, DE). Low methoxyl pectins (hereinafter referred to as LM pectins) have a degree of methoxylation (hereinafter referred to as DM) of less than 50% and high methoxyl pectins (hereinafter referred to as HM pectins) have a DM of greater than 50%. In addition, the degree of amidation (hereinafter referred as DA) indicates the percentage of carboxyl groups converted to the amide. In the presence of metal ions such as calcium ions, pectin chains may cross-link more readily to form a more cohesive gel. The rigidity or strength of the gel and the amount of metal ions required to form the gel, are dependent on the DM and DA percentages for a particular pectin. HM pectins may form gels in the absence of metal ions such as calcium ions due to hydrogen bonding between the pectin chains. LM pectins can be strengthened by the addition of calcium or other divalent or polyvalent metal ions.

Raft forming products comprising pectins as the gel forming ingredient are believed to be particularly suitable for relieving distress caused by gastric reflux for the following reasons. Pectin structure is largely independent of pH. The properties of pectins can be modified by selection of DM or DA values. Pectins break down in the higher pH of the lower intestine to their constituent galactopyransoyluronic acid monosaccharides which are easily absorbed. Pectins form a viscous liquid in the mouth which due to the good lipid binding properties of pectins will coat the lining of the oesophagus and stomach easily when swallowed, thus providing further protection against the effect of gastric reflux. Pectins are readily available from a common source of supply such as discarded plant material like citrus peel or apple pomace.

EP 0286085 (Farma Foods) describes a solid dosage form antacid composition containing pectin as a raft forming component for treatment of gastric reflux. In order to obtain a stable long lasting gel in the stomach, this document teaches use of either a buffering agent or a pectin with a extremely low DM of less than 15%. In addition the composition is directed towards long lasting neutralisation of the stomach contents in which the buffer and an antacid component are released from the gel gradually. These systems are only suitable for powder or tablet presentation, since satisfactory liquid preparations cannot be prepared due to the high percentage of solids and the sensitivity of the system to sparingly soluble metal ions.

Scand. J. Gastroenterol., 1988, 23, p920-24, N Washington et al; describes a study which uses a pectin-casein mixture as the gelling agent in an anti-reflux formulation, comprising caesin with 15% pectin (w/w), 7% potassium bicarbonate (w/w) and 19% magnesium carbonate (w/w). The term 'w/w' as used herein indicates the percentage by mass of an ingredient per total mass of a composition. The casein acts as a buffering component to increase the lifetime of the gel by stabilising the pH in the immediate proximity of the gel.

Rev. Med. Chir. Soc. Med. Nat. lasi 1985 89 (2) p233-6 G Stanciu et al; describes a study comparing a sodium alginate composition with a pectin composition for treatment of gastric reflux. Both compositions are suspensions and the alginate or pectin component are both described as a 'suspension agent having a neutralising effect'. The compositions further comprise an antacid mixture consisting of sodium bicarbonate, aluminium hydroxide and magnesia but no calcium. Both compositions are said to act by 'spreading as a thin film on the stomach acid contents'. The pectin composition disclosed could not form a stable viscous cohesive foamed gel raft, and this paper teaches away from a raft forming product.

Anti-reflux gel raft forming agents act as a barrier to reflux or are refluxed in preference to the acidic gastric juices. These effects are entirely mechanical in nature and therefore antacids are not an essential component to prevent gastric reflux. Formation of a cohesive stable floating raft is dependent on the amount of acid present in the gastric environment both to react with the pectin to form a gel and to generate enough gas to foam the gel to lower its bulk density to enable it to float. Therefore too much antacid (greater than about 10% w/v) in a raft formulation may compete with the acid locally to such an extent that raft forming ability of the formulation is reduced and hence its effectiveness in protecting against gastric reflux is also impaired. The speed of raft formation may also be reduced which delays onset of relief of symptoms. The term 'w/v' as used herein indicates the percentage by mass of an ingredient (measured in grammes) per total volume of a liquid composition (measured in millilitres).

Addition of a buffering component may also compete with the stomach acid and reduce the raft forming ability of the composition or the rapidity of raft formation. Buffering components are added to increase the stability of the raft over longer periods. Their disadvantage is that there is a danger that the rafts thus formed may be too stable, and would not break down easily causing a blockage. As used herein a buffering component is defined to be a component which when present in solution in a suitable amount acts as a reservoir of or for hydrogen ions such that over a wide range of diluents the pH of the resultant solution is practically unchanged and will resist a change of pH on addition of significant amounts of acid and alkali.

Therefore it would be advantageous if a composition could be produced using pectins as the raft forming ingredient, the composition having a simple composition without a buffering component, using pectins from readily available natural sources, and rapidly forming a cohesive gel in the stomach, but not blocking the gastrointestinal tract, and instead breaking up easily in the higher pH of the small intestine.

A problem with liquid preparations containing pectin and insoluble metal salts such as calcium carbonate or calcium phosphate, is that free metal ions are able to cross-link pectin chains causing the liquid preparation to form a gel over time at normal storage temperatures, that is, from 50 °C down to refrigeration temperatures.

It is therefore an object of the invention to provide a liquid preparation that can be stored for long periods without forming a gel and which has some or all of the advantageous properties described above and/or which overcomes some or all of the problems identified above.

It would be further advantageous if such solid pectin raft forming compositions additionally comprised one or more further ingredients to aid patient compliance. Such further ingredients might comprise any of the following: agents which may help adjust, directly or indirectly, stomach pH to physiologically normal levels (for example antacids, proton pump inhibitors and/or H₂ receptor antagonists); anti-infective agents (for example antbiotics such as those effective against Helicobacter pylori either alone or as part of a combination therapy); agents which may act on neurotransmitters (for example anti-cholinergic agents which may block parasympathetically mediated secretory response and increased motility and/or local anaesthetics which may reduce pain and discomfort); anti-emetic agents which may act directly to eliminate or reduce stomach irritation, or indirectly to supress or reduce the reflux to vomit; and/or cytoprotectant agents which may act to enhance the secretion of protective mucus and/or bicarbonate, and/or provide a physical barrier to protect cells within the stomach and small intestine. A preferred aspect of the present invention aims to provide compositions with such further ingredients, which have improved patient compliance over known compositions.

According to the invention there is provided a pharmaceutical composition in liquid form which comprises as a gel raft-forming agent, a pectin, or a pharmaceutically acceptable salt thereof; a pharmaceutically acceptable metal ion component; one or more substances capable of producing a pharmaceutically acceptable gas at the physiological pH normally present in the stomach; the composition forming a raft in a gastric environment; in which the metal ion component is coated with a material to prevent the composition from forming a raft in a non-gastric environment.

By "gastric-environment" it is meant an environment in which the pH is acidic, like the normal physiological pH of the stomach.

"Stable gel raft" as used herein means a cohesive gel structure which forms a physical barrier to gastric reflux.

Preferably the liquid composition of the present invention comprises less than about 20% of solid material by weight of the composition.

The coating material is thought to prevent gel formation by inhibiting the release of free metal ions which can cross-link pectin chains causing the liquid formulation to form a gel over time. Any coating material which inhibits the release of free metal ions should confer non-gel forming properties on the liquid formulation under normal storage conditions.

Preferably, the coating material is hydrophobic and is conveniently a stearate, a silicone, a fat, or an acid disruptable polymer.

A particularly preferred metal ion component and coating material is calcium carbonate with calcium stearate over calcium carbonate BP. Such a material is sold under the trade name Britomya BSH.

The inventor has found that use of a coated metal salt such as Britomya BSH, imparts thixotropic properties to provide a liquid product which does not form a gel and remains stable in a container with storage time and temperature variations.

Preferably the pectin is present in an amount of from about 0.5% to 10%, more preferably from about 1.5% to about 3.5% by weight of the composition.

Preferably, the pectin is a low DM pectin having a degree of methoxylation (DM) of from about 20% to about 50%, more preferably from about 30% to about 40%, and most preferably from about 35% to 40%. When the pectin has a low DM the liquid composition of the invention does not need to contain a buffering component, and this is desirable, as mentioned previously.

Preferably the degree of amidation (DA) of the pectin is from about 3% to about 23%.

Preferably the gas producing substance comprises one or more pharmaceutically acceptable salts selected from bicarbonate and carbonate salts and the pharmaceutically acceptable gas comprises carbon dioxide. More preferably, the gas producing substance is selected from potassium bicarbonate and calcium carbonate.

The gas producing substance may be present in an amount from about 0.1% to about 10%, and preferably from about 1% to about 3%, by weight of the composition. Potassium bicarbonate is a favoured gas producing substance.

Advantageously, the gas producing substance comprises two components, a first fast-acting component (e.g. KHCO₃) to generate the initial bubbles which are trapped in the pectin gel as it is formed, to create a foam, and a second slow-acting component (e.g. CaCO₃) which produces gas over a longer period of time to refresh leakage of the gas entrapped in the gel.

The metal ions aid cross-linkage of the linear pectin chains to form a more cohesive gel. The metal ion component, which may be the same or different from the gas producing substance, may be present in an amount of from about 0.01% to about 5%, and preferably from about 0.1% to about 2.5%, by weight of the composition. Preferably the metal ion component comprises calcium ions which produce a raft which is particularly cohesive and thick.

The composition may additionally comprise a suitable suspending agent for example a carbomer, preferably in an amount from about 0.1% to about 5%, more preferably about 0.5% by weight of the composition.

Preferably the composition further comprises one or more additional ingredients selected from: one or more antacids, one or more antibiotics, one or more anti-cholinergic agents, one or more anti-emetic agents, one or more H₂ receptor antagonists, one or more local anaesthetics, one or more proton pump inhibitors and any suitable and compatible mixtures thereof.

Preferably the antacid agent is present in an amount of from about 0.3% to 10%, preferably about 0.5% to about 4%, more preferably from about 0.5% to about 3% by weight of the composition. Preferably the antacid comprises an aluminium magnesium hydroxy sulphate, more preferably of formula AlMg₁₀(OH)₃₁(SO₄)₂ x H₂0 (also known as magaldrate).

Preferably the antibiotic is present in an amount of from about 0.01% to about 10% of the composition. Advantageously the antibiotic, which may comprise one or more components, (such as in triple or dual therapies) is effective against Helicobacter pylori. A favoured antibiotic comprises: (2S,5R,6R)-6-[(R)-(-)-2-amino-2-(p-hydroxyphenyl)- acetamido]-3,3-dimethyl-7-oxo-4-thia-1-azabicyclo[3.2.0]heptane-2-carboxylic acid trihydrate (the INN [international non-propriety name] of which is amoxicilline and the BAN [British approved name] of which is amoxycillin).

Preferably the anti-cholinergic agent is present in an amount from about 0.01% to about 2% by weight of the composition. A favoured anti-cholinergic agent comprises (cyclohexyl)-1-carboxylic acid, 2-(diethylamino)ethyl ester hydrochloride (the INN and BAN of which is dicyclomine hydrochloride).

Preferably the anti-emetic agent is present in an amount from about 0.01% to about 2% by weight of the composition. A favoured anti-emetic agent comprises 4-amino-5-chloro-N-[2-(diethylamino)ethyl]-2-methoxybenzamide (the BAN and INN of which is metoclopramide).

Preferably the cytoprotectant is present in an amount of from about 0.3% to about 10% by weight of the composition. A favoured cytoprotectant is tri-potassium di-citrato bismuthate.

Preferably the H₂ receptor antagonist is present in an amount of from about 0.01% to about 3%, most preferably from about 0.1 to about 1.5% by weight of the composition. A favoured H₂ receptor antagonist comprises:N"-cyano-N-methyl-N'-[2-{5-methyl-1H-imidazol-4-yl)methyl}thio]-ethy guanidine (the INN and BAN of which is cimetidine) and/or N-(2-*{[(*-{-5-[(dimethylamino)methyl]-2-furanyl}methyl)thio/ethyl})-N-methyl-2-nitro-1,1-ethene-diamine (the INN and BAN of which is ranitidine).

Preferably the local anaesthetic is present in an amount from about 0.01% to about 2% by weight of the composition. A favoured local anaesthetic comprises 2,2'-[(2-hydroxyethyl)-imino]bis[N-(1,1-dimethyl-2-phenylethyl)-N-methyl-acetamide (the INN of which is oxethazaine).

Preferably the proton pump inhibitor is present in an amount of from from about 0.01% to about 3% by weight of the composition. A favoured proton pump inhibitor comprises 5-methoxy-2-{[4-methoxy-2-pyrindyl)methyl]suphinyl}-benzimazole (the INN and BAN of which is omeprazole).

The precise amount of the further ingredients specified herein which may be orally administered to a patient as part of the compostions of the present invention will depend on a number of factors, for example the severity of the condition, the patient being treated and the age and past medical history of the patient, and will lie within the sound discretion of any administering physician, pharmacist and/or other medical practitioner. For example a daily dose of the preferred further ingredients specified herein, (given in a single dose or in divided doses at one or more times during the day) which might be generally suitable for administration to adult human beings and generally therapeutically and/or prophylactically effective may comprise the following for each preferred ingredient: magaldrate from about 100 mg to about 500 mg; amoxycillin from about 100 mg to about 500 mg; dicyclomine hydrochloride from about 1 mg to about 60 mg; metoclopramide from about 5 mg to about 60 mg; tri-potassium di-citrato bismuthate from about 100 mg to about 500 mg; cimetidine from about 50 mg to about 500 mg; ranitidine from about 50 mg to about 500 mg; oxethazaine from about 5 mg to about 60 mg; and omeprazole from about 5 mg to about 60 mg.

The liquid compositions of the present invention are for oral administration and may be prepared by any method known to those skilled in the art to formulate such compositions. For example any pharmacologically active ingredients may be brought into association with suitable inert diluents, carriers and/or any other optional ingredients (for example those described herein). The ingredients comprising the compositions of the present invention may be uniformly and/or intimately admixed. It will be appreciated by those skilled in the art that if the composition contains large amounts of excipients in relation to any active ingredients, repeated conventional mixing operations may be required to distribute the active ingredient evenly and/or homogenously throughout the composition. Compositions of the invention may also be formulated in a manner known to those skilled in the art, to give a controlled release, for example rapid release or sustained release, of any active ingredients. Pharmaceutically acceptable diluents and/or carriers suitable for use in liquid compositions are well known in the art of pharmacy. The excipients used in the preparation of the compositions of the present invention are the excipients known in the pharmacist's art.

The liquid compositions of the present invention may comprise elixirs, solutions, syrups and/or suspensions in pharmaceutically acceptable media. Pharmaceutically acceptable solvents comprise water, glycol, oils and/or alcohols. Media suitable for preparing syrups and/or suspensions may comprise aqueous media, oily media and/or emulsions in the presence of one or more pharmaceutically acceptable suspending agents (for example aluminium stearate gel, cellulose [such as methylcellulose, hydroxyethylcellulose and/or sodium carboxymethylcellulose], gelatin, hydrogenated fats and/or sorbitol). Suitable oily media may comprise vegetable oils (for example arachis oil and/or sunflower oil), other edible oils (for example almond oil and/or fractionated coconut oil) and/or oily esters (for example esters of glycerine, propylene glycol and/or ethanol).

The liquid compositions of the present invention may further comprise one or more of the following which are pharmaceutically acceptable and compatible with the raft forming pectin: agents which vary osmotic pressure (for example salts), colouring agents, emulsifiers (for example lecithin, sorbitan monooleate and/or acacia), flavouring agents, preservatives, sweetening agents and/or any suitable mixtures thereof.

The liquid compositions of the present invention may also be prepared from dry products (for example granules and/or powders) which are presented for reconstitution with a suitable vehicle (for example those media described above).

The invention will now be described with reference to the following examples which are given by way of illustration. All these examples were prepared by mixing the ingredients together. On contact with 0.1M HCI, these liquid formulations rapidly formed a stable, cohesive gel raft which floated on the surface of the liquid. At alkali pH the gel disintegrated rapidly. The 0.1M HCI solution was used to simulate the physiological pH in a "gastric environment". The examples of the present invention can be contrasted with the following formulation (not within the scope of the present invention) which forms a gel within the container on long term storage. The pectin used in these examples was extracted from lemons pomace (available commercially from Allied Colloids) and had a DM of 37% and a DA of 13%. The Britomya BSH material used in the examples is a coated calcium carbonate with calcium stearate over calcium carbonate BP.

### Comparative formulation

| | |
|---|---|
| Amidated low methoxylated pectin | 2.0% w/v |
| Potassium bicarbonate | 2.5% w/v |
| Calcium carbonate | 0.8% w/v |
| Flavouring | qs |
| Preservatives | qs |
| Distilled water | to 100 |

After storage for 4 to 6 weeks at 22C the above liquid formulation formed a gel within its container and was therefore impossible to use in the prevention of gastric reflux.

### Example 1

| | |
|---|---|
| Amidated low methoxylated pectin | 2.0% w/v |
| Potassium bicarbonate | 2.5% w/v |
| Britomya BSH | 0.8% w/v |
| Carbomer (suspending agent) | 0.5% w/v |
| Flavouring | qs |
| Preservatives | qs |
| Distilled water | to 100% |

Surprisingly, this liquid formulation does not form a gel within its container after 6 months storage.

### Example 2 - including antacid component

| | |
|---|---|
| Magaldrate | 4.0% w/v |
| Amidated low methoxylated pectin | 2.0% w/v |
| Potassium bicarbonate | 2.5% w/v |
| Britomya BSH | 0.8% w/v |
| Carbomer (suspending agent) | 0.5% w/v |
| Flavouring | qs |
| Preservatives | qs |
| Distilled water | to 100% |

### Example 3 - including an H₂ receptor antagonist

| | |
|---|---|
| Cimetidine | 1.0% w/v |
| Amidated low methoxylated pectin | 3.0% w/v |
| Potassium bicarbonate | 2.5% w/v |
| Britomya BSH | 1.6% w/v |
| Carbomer (suspending agent) | 0.5% w/v |
| Flavouring | qs |
| Preservatives | qs |
| Distilled water | to 100% |

Examples 4 to 6 comprise a local anaesthetic:

### Example 4

| | |
|---|---|
| Amidated low methoxylated pectin | 2.0% w/v |
| Potassium bicarbonate | 2.5% w/v |
| Britomya BSH | 0.8% w/v |
| Carbomer (suspending agent) | 0.5% w/v |
| Oxethazaine | 0.5% w/v |
| Flavouring | qs |
| Preservatives | qs |
| Distilled water | to 100% |

Surprisingly, this liquid formulation does not form a gel within its container after 6 months storage.

### Example 5 - including antacid component

| | |
|---|---|
| Magaldrate | 4.0% w/v |
| Amidated low methoxylated pectin | 2.0% w/v |
| Potassium bicarbonate | 2.5% w/v |
| Britomya BSH | 0.8% w/v |
| Carbomer (suspending agent) | 0.5% w/v |
| Oxathazaine | 0.5% w/v |
| Flavouring | qs |
| Preservatives | qs |
| Distilled water | to 100% |

### Example 6 - including an H₂ receptor antagonist

| | |
|---|---|
| Cimetidine | 1.0% w/v |
| Amidated low methoxylated pectin | 3.0% w/v |
| Potassium bicarbonate | 2.5% w/v |
| Britomya BSH | 1.6% w/v |
| Carbomer (suspending agent) | 0.5% w/v |
| Oxethazaine | 0.5% w/v |
| Flavouring | qs |
| Preservatives | qs |
| Distilled water | to 100% |

Examples 7 to 9 comprise an anti-cholingeric agent:

### Example 7

| | |
|---|---|
| Amidated low methoxylated pectin | 2.0% w/v |
| Potassium bicarbonate | 2.5% w/v |
| Britomya BSH | 0.8% w/v |
| Carbomer (suspending agent) | 0.5% w/v |
| Dicyclomine hydrochloride | 0.5% w/v |
| Flavouring | qs |
| Preservatives | qs |
| Distilled water | to 100% |

Surprisingly, this liquid formulation does not form a gel within its container after 6 months storage.

### Example 8 - including antacid component

| | |
|---|---|
| Magaldrate | 4.0% w/v |
| Amidated low methoxylated pectin | 2.0% w/v |
| Potassium bicarbonate | 2.5% w/v |
| Britomya BSH | 0.8% w/v |
| Carbomer (suspending agent) | 0.5% w/v |
| Dicyclomine hydrochloride | 0.5% w/v |
| Flavouring | qs |
| Preservatives | qs. |
| Distilled water | to 100% |

### Example 9 - including an H₂ receptor antagonist

| | |
|---|---|
| Cimetidine | 1.0% w/v |
| Amidated low methoxylated pectin | 3.0% w/v |
| Potassium bicarbonate | 2.5% w/v |
| Britomya BSH | 1.6% w/v |
| Carbomer (suspending agent) | 0.5% w/v |
| Dicyclomine hydrochloride | 0.5% w/v |
| Flavouring | qs |
| Preservatives | qs |
| Distilled water | to 100% |

Examples 10 to 12 comprise an anti-emetic agent:

### Example 10

| | |
|---|---|
| Amidated low methoxylated pectin | 2.0% w/v |
| Potassium bicarbonate | 2.5% w/v |
| Britomya BSH | 0.8% w/v |
| Carbomer (suspending agent) | 0.5% w/v |
| Metoclopramide | 0.5% w/v |
| Flavouring | qs |
| Preservatives | qs |
| Distilled water | to 100% |

Surprisingly, this liquid formulation does not form a gel within its container after 6 months storage.

### Example 11 - including antacid component

| | |
|---|---|
| Magaldrate | 4.0% w/v |
| Amidated low methoxylated pectin | 2.0% w/v |
| Potassium bicarbonate | 2.5% w/v |
| Britomya BSH | 0.8% w/v |
| Carbomer (suspending agent) | 0.5% w/v |
| Metocyclopramide | 0.5% w/v |
| Flavouring | qs |
| Preservatives | qs |
| Distilled water | to 100% |

### Example 12 - including an H₂ receptor antagonist

| | |
|---|---|
| Cimetidine | 1.0% w/v |
| Amidated low methoxylated pectin | 3.0% w/v |
| Potassium bicarbonate | 2.5% w/v |
| Britomya BSH | 1.6% w/v |
| Carbomer (suspending agent) | 0.5% w/v |
| Metoclopramide | 0.5% w/v |
| Flavouring | qs |
| Preservatives | qs |
| Distilled water | to 100% |

## Claims

1. A pharmaceutical composition in liquid form which comprises as a gel raft-forming agent, a pectin, or a pharmaceutically acceptable salt thereof; a pharmaceutically acceptable metal ion component; one or more substances capable of producing a pharmaceutically acceptable gas at the physiological pH normally present in the stomach; the composition forming a gel raft in a gastric environment; in which the metal ion component is coated with a material to prevent the composition from forming a gel raft in a non-gastric environment.

2. A composition as claimed in claim 1, in which the coating material is hydrophobic.

3. A composition as claimed in any preceding claim, in which the coating material is selected from a stearate, a silicone, a fat, or an acid disruptable polymer.

4. A composition as claimed in any preceding claim, in which the coating material is calcium stearate.

5. A composition as claimed in any preceding claim, in which the metal ion component is selected from one or more of calcium, magnesium and aluminium ions.

6. A composition as claimed in any preceding claim, in which the metal ion is calcium ion.

7. A composition as claimed in any preceding claim, in which the pectin has a low degree of methoxylation.

8. A composition as claimed in any preceding claim, in which the pectin has a degree of methoxylation of from about 30% to 40%.

9. A composition as claimed in any preceding claim, in which the coated metal ion component is present in an amount of from about 0.01% to about 10% by weight of the composition.

10. A composition as claimed in any preceding claim, in which the pectin is present in an amount of from about 0.5% to about 10% by weight of the composition.

11. A composition as claimed in any preceding claim, which further comprises a suitable suspending agent.

12. A composition as claimed in any preceding claim, which further comprises one or more additional ingredients selected from: one or more antacid agents, one or more antibiotics, one or more anti-cholinergic agents, one or more anti-emetic agents, one or more cytoprotectants, one or more H₂ receptor antagonists, one or more local anaesthetics, one or more proton pump inhibitors and any suitable and compatible mixtures thereof.

13. A composition as claimed in claim 12, in which the further ingredient or ingredients, if present, are selected from one or more of: an antacid comprising magaldrate, an antibiotic comprising amoxycillin, an anti-cholingeric agent comprising dicyclomine hydrochloride, an anti-emetic agent comprising metochlopramide, a cytoprotectant comprising tri-potassium di-citrato bismuthate, a H₂ receptor antagonist comprising cimetidine and/or ranitidine, a local anaesthetic comprising oxethazaine, a proton pump inhibitor comprising omeprazole and any suitable and compatible mixtures thereof.

## Patentansprüche

1. Pharmazeutische Zubereitung in flüssiger Form, die als ein einen Gel-Schwimmkörper (raft) bildendes Mittel ein Pektin oder ein pharmazeutisch annehmbares Salz desselben, eine pharmazeutisch annehmbare Metall-Ionenkomponente, eine oder mehrere Substanzen enthält, die bei dem normalerweise im Magen vorliegenden, physiologischen pH ein pharmazeutisch annehmbares Gas erzeugen können, wobei die Zubereitung einen Gel-Schwimmkörper (raft) in einer gastrischen Umgebung bildet, wobei die Metall-Ionenkomponente mit einem Material beschichtet ist um zu verhindern, dass die Zubereitung in einer nicht-gastrischen Umgebung einen Gel-Schwimmkörper bildet.

2. Zubereitung gemäß Anspruch 1, worin das Beschichtungsmaterial hydrophob ist.

3. Zubereitung gemäß einem der vorstehenden Ansprüche, worin das Beschichtungsmaterial ausgewählt ist unter einem Stearat, einem Silikon, einem Fett oder einem Säure zersetzlichen Polymer.

4. Zubereitung gemäß einem der vorstehenden Ansprüche, worin das Beschichtungsmaterial Calciumstearat ist.

5. Zubereitung gemäß einem der vorstehenden Ansprüche, worin die Metall-Ionenkomponente ausgewählt ist unter einem oder mehreren von Calcium-, Magnesium- und Aluminiumionen.

6. Zubereitung gemäß einem der vorstehenden Ansprüche, worin das Metallion ein Calciumion ist.

7. Zubereitung gemäß einem der vorstehenden Ansprüche, worin das Pektin einen geringen Methoxylierungsgrad aufweist.

8. Zubereitung gemäß einem der vorstehenden Ansprüche, worin das Pektin einen Methoxylierungsgrad von etwa 30 % bis 40 % aufweist.

9. Zubereitung gemäß einem der vorstehenden Ansprüche, worin die beschichtete Metall-Ionenkomponente in einer Menge von etwa 0,01 % bis etwa 10 % in Gewicht der Zubereitung vorliegt.

10. Zubereitung gemäß einem der vorstehenden Ansprüche, worin das Pektin in einer Menge von etwa 0,5 % bis etwa 10 % in Gewicht der Zubereitung vorliegt.

11. Zubereitung gemäß einem der vorstehenden Ansprüche, die zusätzlich ein geeignetes Suspendiermittel enthält.

12. Zubereitung gemäß einem der vorstehenden Ansprüche, die zusätzlich einen oder mehrere zusätzliche Bestandteile enthält ausgewählt unter: einem oder mehreren antaciden Mitteln, einem oder mehreren Antibiotika, einem oder mehreren anticholinergen Mitteln, einem oder mehreren antiemetischen Mitteln, einem oder mehreren Zellschutzmitteln (cytoprotectants), einem oder mehreren H₂-Rezeptor-Antagonisten, einem oder mehreren Lokalanästhetika, einem oder mehreren Inhibitoren der Protonenpumpe und jeder geeigneten und verträglichen Mischung derselben.

13. Zubereitung gemäß Anspruch 12, in der der oder die weite ren Bestandteile, falls vorhanden, ausgewählt sind unter einem oder mehreren aus: einem Magaldrat umfassenden antaciden Mittel, einem Anoxycillin umfassenden Antibiotikum, einem Dicyclominhydrochlorid umfassenden anticholinergen Mittel, einem Metochlopramid umfassenden antiemetischen Mittel, einem Trikalium-di-citrato-bismuthat umfassenden Zellschutzmittel, einem Cimetidin und/oder Ranitidin umfassenden H₂-Rezeptor-Antagonisten, einem Oxethazain umfassenden Lokalanästhetikum, einem Omeprazol umfassenden Inhibitor der Protonenpumpe und jeder geeigneten und verträglichen Mischung derselben.

## Revendications

1. Composition pharmaceutique sous forme liquide qui comprend, comme agent formant une nacelle de gel, une pectine ou un de ses sels pharmaceutiquement acceptables ; un constituant renfermant un ion métallique pharmaceutiquement acceptable ; une ou plusieurs substances capables de produire un gaz pharmaceutiquement acceptable au pH physiologique normalement présent dans l'estomac ; la composition formant une nacelle de gel dans un environnement gastrique ; dans laquelle le constituant renfermant un ion métallique est enrobé d'une matière pour empêcher la composition de former une nacelle de gel dans un environnement non gastrique.

2. Composition suivant la revendication 1, dans laquelle la matière d'enrobage est hydrophobe.

3. Composition suivant l'une quelconque des revendications précédentes, dans laquelle la matière d'enrobage est choisie entre un stéarate, une silicone, une graisse ou un polymère dissociable en milieu acide.

4. Composition suivant l'une quelconque des revendications précédentes, dans laquelle la matière d'enrobage est le stéarate de calcium.

5. Composition suivant l'une quelconque des revendications précédentes, dans laquelle le constituant renfermant un ion métallique choisi consiste en un ou plusieurs des ions calcium, magnésium et aluminium.

6. Composition suivant l'une quelconque des revendications précédentes, dans laquelle l'ion métallique est l'ion calcium.

7. Composition suivant l'une quelconque des revendications précédentes, dans laquelle la pectine a un faible degré de méthoxylation.

8. Composition suivant l'une quelconque des revendications précédentes, dans laquelle la pectine a un degré de méthoxylation d'environ 30 % à 40 %.

9. Composition suivant l'une quelconque des revendications précédentes, dans laquelle le constituant renfermant un ion métallique enrobé est présent en une quantité d'environ 0,01 % à environ 10 % en poids de la composition.

10. Composition suivant l'une quelconque des revendications précédentes, dans laquelle la pectine est présente en une quantité d'environ 0,5 % à environ 10 % en poids de la composition.

11. Composition suivant l'une quelconque des revendications précédentes, qui comprend en outre un agent de mise en suspension convenable.

12. Composition suivant l'une quelconque des revendications précédentes, qui comprend en outre un ou plusieurs ingrédients supplémentaires choisis entre : un ou plusieurs antacides, un ou plusieurs antibiotiques, un ou plusieurs agents anti-cholinergiques, un ou plusieurs agents anti-émétiques, un ou plusieurs cytoprotecteurs, un ou plusieurs antagonistes des récepteurs H₂, un ou plusieurs anesthésiques locaux, un ou plusieurs inhibiteurs de la pompe à protons et n'importe lesquels de leurs mélanges convenables et compatibles.

13. Composition suivant la revendication 12, dans laquelle le ou les ingrédients supplémentaires, s'ils sont présents, sont choisis de manière à consister en un ou plusieurs des suivants : un antacide comprenant le magaldrate, un antibiotique comprenant l'amoxycilline, un agent anti-cholinergique comprenant le chlorhydrate de dicyclomine, un agent anti-émétique comprenant le métochlopramide, un agent cytoprotecteur comprenant le dicitrato-bismuthate tripotassique, un antagoniste des récepteurs H₂ comprenant la cimétidine et/ou la ranitidine, un anesthésique local comprenant l'oxéthazaïne, un inhibiteur de la pompe à protons comprenant l'oméprazole et n'importe lequel de leurs mélanges convenables et compatibles.
